# EUROPÄISCHE PATENTSCHRIFT

(11) **EP 3 962 575 B1**
(45) Veröffentlichungstag und Bekanntmachung des Hinweises auf die Patenterteilung: **17.07.2024**
(21) Anmeldenummer: 20720004.9
(22) Anmeldetag: 16.04.2020
(51) Int. Cl.: A61M 39/10, A61J 1/20

(54) **MEDIZINISCHE FLUIDÜBERTRAGUNGSVORRICHTUNG**
MEDICAL FLUID TRANSFER APPARATUS
DISPOSITIF DE TRANSMISSION DE FLUIDE MÉDICAL

(30) Priorität: 29.04.2019 DE 102019206126
(43) Veröffentlichungstag der Anmeldung: 09.03.2022
(73) Patentinhaber: B. Braun Melsungen AG, 34212 Melsungen (DE)
(72) Erfinder: BERG, Karl Martin, 34212 Melsungen (DE); FISCHER, Nathanael, 36282 Hauneck (DE); KOPP, Florin, 26419 Schortens (DE); KRUG-SAUER, Konstantin, 34281 Gudensberg (DE)
(74) Vertreter: Patentanwälte Ruff, Wilhelm, Beier, Dauster & Partner mbB
(86) Internationale Anmeldenummer: PCT/EP2020/060678
(87) Internationale Veröffentlichungsnummer: WO 2020/221594

(56) Entgegenhaltungen:
- DE-A1- 2 652 754
- US-A- 4 699 615
- US-A- 5 697 919
- US-A1- 2013 228 239
- US-A1- 2017 333 286
- US-B1- 6 213 981

## Beschreibung

Die Erfindung betrifft eine medizinische Fluidübertragungsvorrichtung zum fluiddicht abgeschlossenen Übertragen einer gesundheitsgefährdenden Medikamentenflüssigkeit, aufweisend einen ersten Konnektorabschnitt, der einen ersten Durchlass aufweist und zur fluiddichten Verbindung mit einer medizinischen Spritze vorgesehen ist, einen zweiten Konnektorabschnitt, der einen zweiten Durchlass aufweist und zur fluiddichten Verbindung mit einem medizinischen Flüssigkeitsbehälter vorgesehen ist, einen zwischen dem ersten Durchlass und dem zweiten Durchlass erstreckten Fluidkanal, der - in einem mit der Spritze und dem Flüssigkeitsbehälter verbundenen Zustand der Fluidübertragungsvorrichtung - eine fluidleitende Übertragung der Medikamentenflüssigkeit zwischen der Spritze und dem Flüssigkeitsbehälter gestattet, und einen Ausgleichsbehälter mit einem fluiddichten und volumenveränderlichen Ausgleichsvolumen, das - im verbundenen Zustand der Fluidübertragungsvorrichtung - fluidleitend mit dem Flüssigkeitsbehälter verbunden ist, und das zur Aufnahme eines bei der Übertragung der Medikamentenflüssigkeit aus dem Flüssigkeitsbehälter verdrängten Gasvolumens eingerichtet ist.

Eine derartige medizinische Fluidübertragungsvorrichtung ist im Bereich der Medizintechnik allgemein bekannt und kann auch als Transfersystem oder Closed System Transfer Device (CSTD) bezeichnet werden. Solche Fluidübertragungsvorrichtungen sind zur Verwendung bei der Zubereitung applikationsfertiger CMR-Medikamentenflüssigkeiten vorgesehen, wobei mit der Abkürzung CMR (Cancerogen Mutagen Reprotoxic) üblicherweise krebserzeugende, erbgutverändernde und fruchtbarkeitsgefährdende Medikamente bezeichnet werden.

Bei der Zubereitung einer solchen Medikamentenflüssigkeit wird diese üblicherweise mittels einer medizinischen Spritze in einen medizinischen Flüssigkeitsbehälter eingebracht oder aus diesem entnommen. Um hierbei eine Gesundheitsgefährdung durch verdrängungsbedingt aus dem Flüssigkeitsbehälter austretende toxische Gase zu vermeiden, soll die bekannte Fluidübertragungsvorrichtung eine fluiddicht bzw. hermetisch abgeschlossene Übertragung der gesundheitsgefährdenden Medikamentenflüssigkeit zwischen der Spritze und dem Flüssigkeitsbehälter ermöglichen.

Hierzu weist die bekannte Fluidübertragungsvorrichtung einen ersten Konnektorabschnitt mit einem ersten Durchlass und einen zweiten Konnektorabschnitt mit einem zweiten Durchlass auf. Der erste Konnektorabschnitt ist zur fluiddichten Verbindung mit der medizinischen Spritze vorgesehen. Der zweite Konnektorabschnitt ist zur fluiddichten Verbindung mit dem medizinischen Flüssigkeitsbehälter vorgesehen. Der erste Durchlass und der zweite Durchlass sind mittels eines Fluidkanals fluidleitend miteinander verbunden. Außerdem ist ein Ausgleichsbehälter mit einem fluiddichten und volumenveränderlichen Ausgleichsvolumen vorgesehen. In einem betriebsfertig mit der Spritze und dem Flüssigkeitsbehälter verbundenen Zustand der Fluidübertragungsvorrichtung ist das Ausgleichsvolumen fluidleitend mit dem Flüssigkeitsbehälter verbunden. Das Ausgleichsvolumen dient einer Aufnahme eines bei der Übertragung der Medikamentenflüssigkeit von der Spritze in den Flüssigkeitsbehälter aus dem Flüssigkeitsbehälter verdrängten Gasvolumens. Bei der bekannten Fluidübertragungsvorrichtung ist der Ausgleichsbehälter eine im Bereich des zweiten Konnektorabschnitts abragend angeordnete elastische Blase, wobei das Ausgleichsvolumen der Blase abseits des Fluidkanals und gesondert von diesem ausgebildet ist. Aus dem Stand der Technik sind beispielsweise die DE 2652754 A1, die US 2017/0333286 A1, sowie die US 2013/0228239 A1 bekannt, welche alle jeweils eine medizinische Fluidübertragungsvorrichtung offenbaren, welche ein volumenveränderliches Ausgleichsvolumen umfasst.

Aufgabe der Erfindung ist es, eine medizinische Fluidübertragungsvorrichtung der eingangs genannten Art bereitzustellen, die einen vereinfachten Aufbau aufweist und gleichzeitig eine verbesserte Handhabung ermöglicht. Diese Aufgabe wird mittels der in den beiliegenden Ansprüchen definierten Vorrichtung gelöst.

Hierbei wird der erste Durchlass und der zweite Durchlass über das Ausgleichsvolumen fluidleitend miteinander verbunden, wobei das Ausgleichsvolumen einen Fluidkanalabschnitt des Fluidkanals bildet. Durch die erfindungsgemäße Lösung kann insbesondere auf eine von dem Fluidkanal gesonderte Anordnung des Ausgleichsvolumens verzichtet werden. Hierdurch kann ein besonders einfacher Aufbau der Fluidübertragungsvorrichtung erreicht werden. Da das Ausgleichsvolumen erfindungsgemäß einen Fluidkanalabschnitt des Fluidkanals bildet, kommt dem Ausgleichsvolumen eine besonders vorteilhafte Mehrfachfunktion zu. Denn zum einen dient das Ausgleichsvolumen zur Aufnahme des aus dem Flüssigkeitsbehälter verdrängten Gasvolumens. Zusätzlich fungiert das Ausgleichsvolumen als fluidführender Abschnitt des Fluidkanals. Diese Mehrfachfunktion des Ausgleichsvolumens ermöglicht insbesondere den erfindungsgemäß besonders einfachen Aufbau der Fluidübertragungsvorrichtung. Zudem kann durch die erfindungsgemäße Lösung insbesondere eine seitlich oder anderweitig von den übrigen Abschnitten und/oder Bauteilen der Fluidübertragungsvorrichtung abragende Anordnung des Ausgleichsbehälters vermieden werden. Hierdurch kann eine vereinfachte Handhabung der Fluidübertragungsvorrichtung bei der Zubereitung der Medikamentenflüssigkeit erreicht werden. Der erste Konnektorabschnitt kann insbesondere einen Luer- oder sonstigen Fluidkonnektor aufweisen, der zur fluiddichten Verbindung mit einem komplementären Luer- bzw. Fluidkonnektor der medizinischen Spritze

eingerichtet ist. Der erste Durchlass kann insbesondere in Form eines Kanals oder eines Lumens durch den ersten Konnektorabschnitt erstreckt sein. Je nach Strömungsrichtung der Medikamentenflüssigkeit bei der Übertragung kann der erste Durchlass als Einlass oder als Auslass der Fluidübertragungsvorrichtung fungieren. Der zweite Konnektorabschnitt kann insbesondere einen Einstechdorn aufweisen, der zum Einstechen in einen hierfür vorgesehenen Abschnitt des Flüssigkeitsbehälters eingerichtet ist. Alternativ kann der zweite Konnektorabschnitt einen Gewinde- oder Steckabschnitt aufweisen, der zur Verbindung mit einem komplementären Gewinde- oder Steckabschnitt des Flüssigkeitsbehälters eingerichtet ist. Der zweite Durchlass kann insbesondere in Form eines Kanals oder eines Lumens durch den zweiten Konnektorabschnitt erstreckt sein. Je nach Strömungsrichtung der Medikamentenflüssigkeit bei der Übertragung kann der zweite Durchlass als Einlass oder als Auslass der Fluidübertragungsvorrichtung fungieren. Der Fluidkanal bildet eine fluidleitende Verbindung zwischen dem ersten Durchlass und dem zweiten Durchlass. In einem betriebsfertig mit der Spritze und dem Flüssigkeitsbehälter verbundenen Zustand der Fluidübertragungsvorrichtung kann die Medikamentenflüssigkeit somit beispielsweise ausgehend von der Spritze über den ersten Durchlass in den Fluidkanal und von dort über den zweiten Durchlass in den Flüssigkeitsbehälter gelangen oder umgekehrt. Da das Ausgleichsvolumen erfindungsgemäß einen Fluidkanalabschnitt des Fluidkanals bildet, wird hierbei auch das Ausgleichsvolumen von der Medikamentenflüssigkeit durchströmt.

In Ausgestaltung der Erfindung sind der erste Konnektorabschnitt und der zweite Konnektorabschnitt an einander gegenüberliegenden Stirnendbereichen des Ausgleichsbehälters angeordnet. Hierdurch kann ein nochmals vereinfachter Aufbau der Fluidübertragungsvorrichtung erreicht werden. Bei dieser Ausgestaltung der Erfindung fungiert der Ausgleichsbehälter demnach auch als tragende und/oder verbindende Struktur, wobei die beiden Konnektorabschnitte mittelbar oder unmittelbar an dem Ausgleichsbehälter festgelegt sein können.

In weiterer Ausgestaltung der Erfindung ist der Ausgleichsbehälter aus einem elastischen Werkstoff gefertigt und/oder weist eine elastische Gestaltgebung auf, wodurch das Ausgleichsvolumen zur Volumenveränderung elastisch expandierbar und/oder elastisch kontrahierbar ist. Als elastischer Werkstoff kann insbesondere ein Elastomer oder ein anderweitiger elastischer Kunststoff gewählt sein. Der elastische Werkstoff weist vorzugsweise gummielastische und/oder weichelastische Eigenschaften auf. Die alternativ oder zusätzlich vorgesehene elastische Gestaltgebung des Ausgleichsbehälters kann insbesondere durch eine dünnwandige Gestaltung einer Behälterwandung erreicht werden. Bei einer solchen Gestaltgebung muss der Ausgleichsbehälter nicht zwingend aus einem elastischen Werkstoff gefertigt sein. Stattdessen kann der Ausgleichsbehälter insbesondere aus einem formstabilen Werkstoff, beispielsweise einem formstabilen Kunststoff oder aus Metall, gefertigt sein. Durch diese Ausgestaltung der Erfindung kann das volumenveränderliche Ausgleichsvolumen mit besonders einfachen konstruktiven Mitteln bereitgestellt werden.

In weiterer Ausgestaltung der Erfindung ist der Ausgleichsbehälter ein Faltenbalg. Vorzugsweise ist der Faltenbalg aus einem elastischen Werkstoff gefertigt. Alternativ kann der Faltenbalg aus einem dünnwandigen formstabilen Werkstoff gefertigt sein. Zur Volumenveränderung des Ausgleichsvolumens ist der Faltenbalg auf grundsätzlich bekannte Weise ziehharmonikaartig axial expandierbar und/oder axial stauchbar.

In weiterer Ausgestaltung der Erfindung weist der Ausgleichsbehälter wenigstens zwei aus einem formstabilen Werkstoff gefertigte Behälterteile auf, die das Ausgleichsvolumen umgrenzen und die zur Volumenänderung des Ausgleichsvolumens zueinander relativbeweglich sind. Die Behälterteile können insbesondere aus einem formstabilen Kunststoff oder aus Metall gefertigt sein. Durch diese Ausgestaltung der Erfindung wird insbesondere einer unbeabsichtigten Beschädigung des Ausgleichsvolumens und somit einer gesundheitsgefährdenden Kontamination der Umgebung mit der Medikamentenflüssigkeit entgegengewirkt. Die wenigstens zwei Behälterteile können insbesondere relativ zueinander linearbeweglich sein. Vorzugsweise sind die wenigstens zwei Behälterteile verschieblich aneinander festgelegt. Zur fluiddichten Abdichtung der wenigstens zwei Behälterteile kann ein gesondertes Dichtelement vorgesehen sein. Alternativ oder zusätzlich können die wenigstens zwei Behälterteile fluiddicht und zueinander beweglich zusammengepasst sein.

In weiterer Ausgestaltung der Erfindung ist der Ausgleichsbehälter zur Übertragung der Medikamentenflüssigkeit zwischen dem ersten Durchlass und dem zweiten Durchlass manuell pumpbeweglich. Bei dieser Ausgestaltung der Erfindung weist der Ausgleichsbehälter demnach eine weitere Funktion auf. Denn neben der Aufnahme des verdrängten Gasvolumens und der Fluidleitung zwischen dem ersten Durchlass und dem zweiten Durchlass dient der Ausgleichsbehälter hier zusätzlich einer Pumpförderung der Medikamentenflüssigkeit zwischen dem ersten Durchlass und dem zweiten Durchlass. Beispielsweise kann die Medikamentenflüssigkeit zunächst mittels der Spritze über den ersten Durchlass in den Fluidkanal abgegeben werden. Demnach befindet sich wenigstens ein Teil der Medikamentenflüssigkeit in dem von dem Ausgleichsvolumen gebildeten Fluidkanalabschnitt. Hiernach kann die Medikamentenflüssigkeit mittels einer entsprechenden manuellen Pumpbetätigung des Ausgleichsbehälters von dem Ausgleichsvolumen weiter in Richtung des zweiten Durchlasses gefördert werden. Um hierbei ein ungewolltes Zurückströmen der Medikamentenflüssigkeit in Richtung des ersten Durchlasses zu vermeiden, kann ein Sperr- oder Rückschlagventil im Bereich des ersten Durchlasses angeordnet sein. Dies ist jedoch nicht zwingend. Die Pumpbeweglichkeit des Ausgleichsbehälters kann insbesondere durch eine elastische Gestaltung desselben und/oder durch zueinander pumpbeweglich verlagerbare formstabile Abschnitte und/oder Bauteile des Ausgleichsbehälters erreicht werden.

In weiterer Ausgestaltung der Erfindung sind mit dem Ausgleichsbehälter wirkverbundene Handhabungsflächen vorgesehen, die zur manuellen Pumpbewegung des Ausgleichsbehälters relativ zueinander verlagerbar sind. Die Handhabungsflächen können insbesondere zur radialen und/oder axialen manuellen Einwirkung auf den Ausgleichsbehälter angeordnet sein. Vorzugsweise sind die Handhabungsflächen an einander gegenüberliegenden Stirnendbereichen des Ausgleichsbehälters angeordnet. Die manuelle Pumpbewegung des Ausgleichsbehälters ist vorzugsweise mittels eines Zusammendrückens der Handhabungsflächen zwischen den Fingern einer Hand bewirkbar. Die Handhabungsflächen können unmittelbar an dem Ausgleichsbehälter ausgebildet sein. Alternativ können die Handhabungsflächen an mit dem Ausgleichsbehälter zusammengefügten Abschnitten und/oder Bauteilen ausgebildet sein.

In weiterer Ausgestaltung der Erfindung ist eine mit dem Ausgleichsbehälter wirkverbundene Federeinrichtung vorgesehen, die eine das Ausgleichsvolumen expandierende Federkraft bewirkt. Die Federeinrichtung dient einer vereinfachten Pumpbeweglichkeit des Ausgleichsbehälters und bewirkt, dass das Ausgleichsvolumen ausgehend von einem manuell zusammengedrückten oder kontrahierten Zustand mittels der Federkraft selbsttätig in einen expandierten Zustand rückstellbar ist. Die Federeinrichtung kann durch eine entsprechende federelastische Gestaltung des Ausgleichsbehälters und/oder durch ein separates Federelement ausgebildet sein. Das Federelement ist vorzugsweise eine Wendelfeder.

In weiterer Ausgestaltung der Erfindung ist ein zwischen einer Sperrstellung und einer Durchflussstellung überführbares erstes Ventil vorgesehen, mittels dessen der erste Durchlass in der Sperrstellung fluiddicht von dem Ausgleichsvolumen getrennt ist und in der Durchflussstellung fluidleitend mit dem Ausgleichsvolumen verbunden ist. Das erste Ventil kann mittels einer manuellen Betätigung oder selbsttätig zwischen der Sperrstellung und der Durchflussstellung überführbar sein. Durch das erste Ventil wird insbesondere einem ungewollten Zurückströmen der Medikamentenflüssigkeit von dem Fluidkanal bzw. dem Ausgleichsvolumen in Richtung des ersten Durchlasses entgegengewirkt. Außerdem kann durch das erste Ventil vermieden werden, dass die Medikamentenflüssigkeit ungewollt durch den ersten Durchlass in das Ausgleichsvolumen bzw. den Fluidkanal gelangt. Hierdurch kann eine nochmals verbesserte Handhabung erreicht werden.

In weiterer Ausgestaltung der Erfindung ist das erste Ventil ein zur manuellen Betätigung eingerichtetes Sperrventil. Demnach ist das Sperrventil manuell zwischen der Sperrstellung und der Durchflussstellung überführbar.

In weiterer Ausgestaltung der Erfindung ist ein zwischen einer Sperrstellung und einer Durchflussstellung überführbares zweites Ventil vorgesehen, mittels dessen das Ausgleichsvolumen in der Sperrstellung fluiddicht von dem zweiten Durchlass getrennt ist und in der Durchflussstellung fluidleitend mit dem zweiten Durchlass verbunden ist. Das zweite Ventil kann mittels einer manuellen Betätigung oder selbsttätig zwischen der Sperrstellung und der Durchflussstellung überführbar sein. Durch das zweite Ventil wird insbesondere vermieden, dass die Medikamentenflüssigkeit ausgehend von dem Ausgleichsvolumen bzw. von dem durch das Ausgleichsvolumen gebildeten Fluidkanalabschnitt in Richtung des zweiten Durchlasses gelangen kann. Außerdem wird durch das zweite Ventil vermieden, dass die Medikamentenflüssigkeit ausgehend von dem Flüssigkeitsbehälter ungewollt in das Ausgleichsvolumen bzw. den Fluidkanalabschnitt gelangen kann. Durch diese Ausgestaltung der Erfindung wird eine nochmals verbesserte Sicherheit bei der Handhabung der Fluidübertragungsvorrichtung erreicht.

In weiterer Ausgestaltung der Erfindung ist das zweite Ventil ein Druckventil, das in Abhängigkeit eines in dem Ausgleichsvolumen anliegenden Drucks selbsttätig zwischen der Sperrstellung und der Durchflussstellung überführbar ist. Der in dem Ausgleichsvolumen anliegende Druck kann insbesondere durch eine Betätigung der medizinischen Spritze bewirkt sein. Alternativ oder zusätzlich kann der Druck durch eine manuelle Pumpbewegung des Ausgleichsbehälters bewirkt sein. Erreicht oder übersteigt der Druck einen konstruktiv festgelegten Grenzdruck des Druckventils, wird dieses selbsttätig von der Sperrstellung in die Durchflussstellung überführt. Umgekehrt kann das Druckventil bei Erreichen oder Unterschreiten des Grenzdrucks selbsttätig von der Durchflussstellung in die Sperrstellung überführt werden.

In weiterer Ausgestaltung der Erfindung nehmen das erste Ventil und das zweite Ventil in einem Auslieferungszustand der Fluidübertragungsvorrichtung jeweils die Sperrstellung ein, und das Ausgleichsvolumen ist mit einem sterilen Gas befüllt. Durch diese Ausgestaltung der Erfindung wird insbesondere einem ungewollten Zusammendrücken oder gar Kollabieren des Ausgleichsbehälters entgegengewirkt. Zu diesem Zweck ist das Ausgleichsvolumen mit dem sterilen Gas befüllt und zudem mittels des ersten und des zweiten Ventils fluiddicht verschlossen. Hierdurch kann eine nochmals verbesserte Handhabung erreicht werden.

In weiterer Ausgestaltung der Erfindung ist eine dem zweiten Konnektorabschnitt zugeordnete Rasteinrichtung vorgesehen, die eine unlösbare Rastverbindung mit dem Medikamentenbehälter gestattet. Mittels der Rasteinrichtung kann eine unlösbare form- und/oder kraftschlüssige Verbindung zwischen der Fluidübertragungsvorrichtung und dem Medikamentenbehälter erreicht werden. Hierdurch wird vermieden, dass die Fluidübertragungsvorrichtung in der Anwendung unbeabsichtigt von dem Medikamentenbehälter getrennt wird. Dies erlaubt eine hinsichtlich ihrer Sicherheit nochmals verbesserte Handhabung der Fluidübertragungsvorrichtung.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Perspektivdarstellung eine Ausführungsform einer erfindungsgemäßen medizinischen Fluidübertragungsvorrichtung in einem mit einem medizinischen Flüssigkeitsbehälter verbundenen Zustand,
- Fig. 2: in schematischer Längsschnittdarstellung die Fluidübertragungsvorrichtung nebst dem Flüssigkeitsbehälter gemäß Fig. 1,
- Fig. 3, 4: jeweils in schematischer Längsschnittdarstellung die Fluidübertragungsvorrichtung und den Flüssigkeitsbehälter nach den Fig. 1 und 2 in unterschiedlichen Zuständen beim Zuführen einer gesundheitsgefährdenden Medikamentenflüssigkeit in den Flüssigkeitsbehälter,
- Fig. 5, 6: jeweils in schematischer Längsschnittdarstellung die Fluidübertragungsvorrichtung und den Flüssigkeitsbehälter nach den Fig. 1 bis 4 in unterschiedlichen Zuständen beim Entnehmen der gesundheitsgefährdenden Medikamentenflüssigkeit aus dem Flüssigkeitsbehälter,
- Fig. 7: in teilweise abgeschnittener schematisch stark vereinfachter Längsschnittdarstellung eine weitere Ausführungsform einer erfindungsgemäßen medizinischen Fluidübertragungsvorrichtung,
- Fig. 8: in schematischer Längsschnittdarstellung ein Behälterteil eines Ausgleichsbehälters der Fluidübertragungsvorrichtung nach Fig. 7,
- Fig. 9: das Behälterteil nach Fig. 8 in einer axial von oben nach unten gerichteten Aufsicht,
- Fig. 10: in schematischer Längsschnittdarstellung ein weiteres Behälterteil des Ausgleichsbehälters der Fluidübertragungsvorrichtung nach Fig. 7 und
- Fig. 11: das weitere Behälterteil nach Fig. 10 in einer schematischen Perspektivdarstellung.

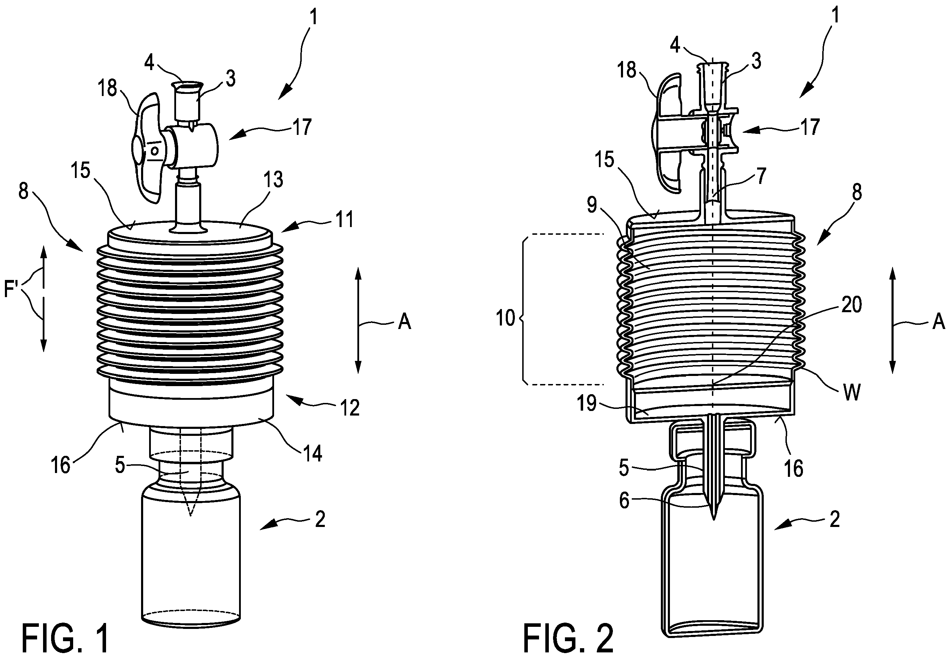

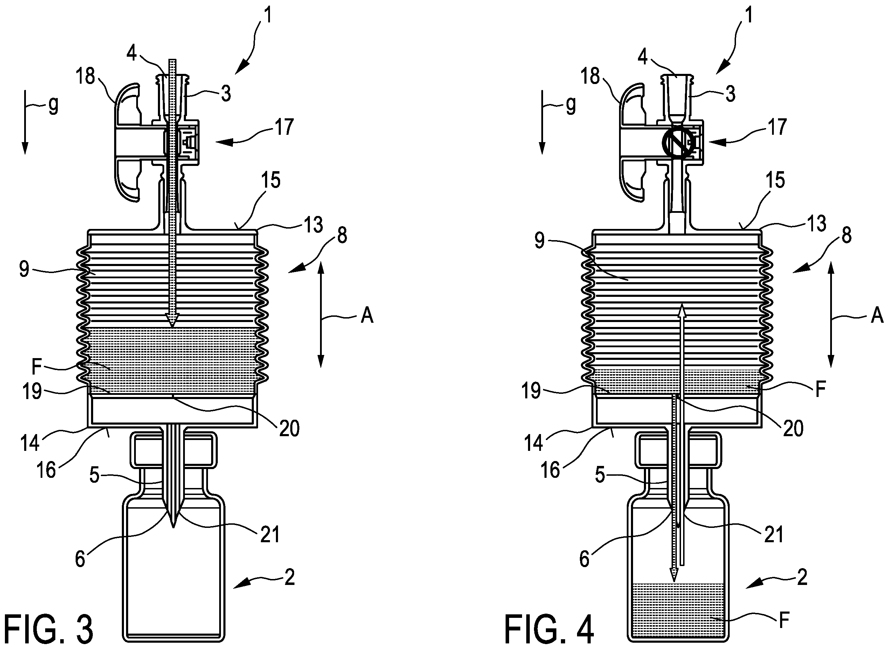

Gemäß den Fig. 1 bis 6 ist eine medizinische Fluidübertragungsvorrichtung 1 zur Verwendung bei einer applikationsfertigen Zubereitung einer gesundheitsgefährdenden Medikamentenflüssigkeit F (Fig. 3) vorgesehen. Die medizinische Fluidübertragungsvorrichtung 1 kann auch als Transfersystem oder Closed System Transfer Device bezeichnet werden. Bei der Medikamentenflüssigkeit F handelt es sich um eine sogenannte CMR-Medikamentenflüssigkeit (Cancerogen Mutagen Reprotoxic), die zur Verwendung bei einer Krebstherapie vorgesehen ist. Die Medikamentenflüssigkeit F kann krebserzeugend, erbgutverändernd und/oder fortpflanzungsgefährdend sein und ist somit als gesundheitsgefährdend einzustufen.

Bei der applikationsfertigen Zubereitung der Medikamentenflüssigkeit F durch medizinisches Personal wird die Medikamentenflüssigkeit F mittels einer zeichnerisch nicht näher dargestellten medizinischen Spritze einem medizinischen Flüssigkeitsbehälter 2 zugeführt und/oder diesem entnommen. Um hierbei eine Gesundheitsgefährdung des medizinischen Personals zu vermeiden, muss eine Kontamination der Umgebung mit der Medikamentenflüssigkeit F vermieden werden. Die Fluidübertragungsvorrichtung 1 dient in erster Linie diesem Zweck.

Die medizinische Fluidübertragungsvorrichtung 1 weist einen ersten Konnektorabschnitt 3 mit einem ersten Durchlass 4 und einen zweiten Konnektorabschnitt 5 mit einem zweiten Durchlass 6 auf.

Der erste Konnektorabschnitt ist vorliegend in Form eines Luer-Lock-Anschlusses 3 ausgebildet, was jedoch nicht zwingend ist. Alternativ kann der erste Konnektorabschnitt 3 auch in Form einer sonstigen im Bereich der Medizintechnik allgemein bekannten Fluidkupplung ausgebildet sein. Zur fluidführenden Verbindung mit der medizinischen Spritze wird der Luer-Lock-Anschluss 3 auf grundsätzlich bekannte Weise mit einem auslassseitig an der Spritze angeordneten komplementären Luer-Lock-Anschluss verbunden.

Der zweite Konnektorabschnitt ist vorliegend in Form eines Einstechdorns 5 ausgebildet, der zur fluiddichten Verbindung mit dem Flüssigkeitsbehälter 2 durch einen hierfür vorgesehenen Abschnitt des Flüssigkeitsbehälters 2 in denselben eingestochen wird. Zu diesem Zweck kann der Flüssigkeitsbehälter 2 beispielsweise einen durchstoßbaren Stopfen, eine Membran oder dergleichen aufweisen. Auch eine solche Gestaltung ist nicht zwingend. Anstelle des Einstechdorns 5 ist insbesondere auch eine Schraub- oder Steckverbindung mit dem Flüssigkeitsbehälter 2 denkbar.

Der erste Durchlass ist in Form eines in Axialrichtung A erstreckten Lumens 4 durch den ersten Konnektorabschnitt 3 erstreckt. Dementsprechend ist der zweite Durchlass in Form eines Lumens 6 in Axialrichtung A durch den Einstechdorn 5 erstreckt.

Zwischen dem ersten Durchlass 4 und dem zweiten Durchlass 6 ist ein Fluidkanal 7 erstreckt, der anhand Fig. 2 schematisch stark vereinfacht durch eine strichlierte Linie zwischen dem ersten Durchlass 4 und dem zweiten Durchlass 6 verdeutlicht sein soll. In einem mit der - zeichnerisch nicht dargestellten - Spritze und dem Flüssigkeitsbehälter 2 verbundenen Zustand der Fluidübertragungsvorrichtung 1 kann die Medikamentenflüssigkeit F von der Spritze in den ersten Durchlass 4 und von dort über den Fluidkanal 7 durch den zweiten Durchlass 6 in den Flüssigkeitsbehälter 2 gelangen und umgekehrt.

Der Flüssigkeitsbehälter ist vorliegend in Form einer Medikamentenflasche 2 ausgebildet, die auch als Ampulle, Phiole oder Vial bezeichnet werden kann. Die Medikamentenflasche 2 kann aus einem formstabilen Werkstoff, wie beispielsweise einem Kunststoff oder Glas, gefertigt sein.

Die Fluidübertragungsvorrichtung 1 weist zudem einen Ausgleichsbehälter 8 mit einem fluiddichten und volumenveränderlichen Ausgleichsvolumen 9 auf. Das Ausgleichsvolumen 9 ist auf noch näher beschriebene Weise fluidleitend mit dem Flüssigkeitsbehälter 2 verbunden und insbesondere zur Aufnahme eines bei der Übertragung der Medikamentenflüssigkeit F aus dem Flüssigkeitsbehälter 2 verdrängten Gasvolumens vorgesehen.

Dabei ist insbesondere anhand Fig. 2 ersichtlich, dass der erste Durchlass 4 und der zweite Durchlass 6 über das Ausgleichsvolumen 9 fluidleitend miteinander verbunden sind, wobei das Ausgleichsvolumen 9 einen Fluidkanalabschnitt 10 des Fluidkanals 7 bildet.

Wird dem Flüssigkeitsbehälter 2 ausgehend von der anhand Fig. 2 ersichtlichen Konfiguration Medikamentenflüssigkeit F mittels der Spritze über den ersten Durchlass 4, den Fluidkanal 7 - und den Fluidkanalabschnitt 10 - und von dort weiter durch den zweiten Durchlass 6 zugeführt, kommt es naturgemäß zu einer Verdrängung eines nicht näher bezeichneten Gasvolumens aus dem Flüssigkeitsbehälter 2. Dieses Gasvolumen kann durch einen Kontakt mit der Medikamentenflüssigkeit F gesundheitsgefährdend kontaminiert sein. Dabei dient das Ausgleichsvolumen 9 einer fluiddichten Aufnahme des Gasvolumens, so dass dieses nicht in die Umgebung abgegeben wird. Zu diesem Zweck ist das Ausgleichsvolumen 9 auf noch näher beschriebene Weise fluidleitend mit dem Flüssigkeitsbehälter 2 verbunden und zudem volumenveränderlich gestaltet. Das heißt bei einem Einströmen des Gasvolumens in das Ausgleichsvolumen 9 vergrößert sich das Ausgleichsvolumen dementsprechend.

Nachfolgend wird näher auf die anhand der Fig. 1 bis 6 ersichtliche spezifische Ausgestaltung der Fluidübertragungsvorrichtung 1 eingegangen. Die in diesem Zusammenhang zusätzlich erläuterten strukturellen und funktionellen Merkmale sind jedoch nicht als zwingend notwendig zu erachten.

Vorliegend ist der Ausgleichsbehälter in Form eines Faltenbalgs 8 ausgebildet. Der Faltenbalg 8 ist aus einem elastischen Werkstoff W gefertigt. Als elastischer Werkstoff W ist vorliegend ein Elastomer gewählt. Durch die vorliegende Werkstoffwahl und die elastische Gestaltgebung als Faltenbalg ist der Ausgleichsbehälter 8 in Axialrichtung A ziehharmonikaartig dehnbar und stauchbar. Das Ausgleichsvolumen 9 ist aufgrund dieser ziehharmonikaartigen Beweglichkeit des Faltenbalgs 8 dementsprechend veränderlich bzw. vergrößerbar und verringerbar. Der Faltenbalg 8 weist eine kreiszylindrische Grundform auf, was jedoch nicht zwingend ist.

Der erste Konnektorabschnitt 3 und der zweite Konnektorabschnitt 5 sind an in Axialrichtung A einander gegenüberliegenden Stirnendbereichen 11, 12 des Faltenbalgs 8 angeordnet. Zu diesem Zweck sind vorliegend zwei aus einem nicht näher bezeichneten formstabilen Werkstoff gefertigte Gehäuseteile 13, 14 vorgesehen. Das erste Gehäuseteil 13 ist einends stirnendseitig fluiddicht in eine axiale Öffnung des Faltenbalgs 8 eingefügt. Dementsprechend ist das zweite Gehäuseteil 14 andernends in eine gegenüberliegende axiale Öffnung des Faltenbalgs 8 fluiddicht eingefügt.

Der Faltenbalg 8 ist in Axialrichtung A manuell pumpbeweglich. Infolge dieser Pumpbeweglichkeit kann die Übertragung der Medikamentenflüssigkeit F zwischen dem ersten Durchlass 4 und dem zweiten Durchlass 6 auf noch näher beschriebene Weise bewirkt und/oder unterstützt sein.

Um eine erleichterte manuelle Pumpbeweglichkeit des Faltenbalgs 8 zu ermöglichen, sind vorliegend eine erste Handhabungsfläche 15 und eine zweite Handhabungsfläche 16 vorgesehen. Die erste Handhabungsfläche 15 ist an dem ersten Gehäuseteil 13 angeordnet und in Axialrichtung A orientiert. Die zweite Handhabungsfläche 16 ist an dem zweiten Gehäuseteil 14 angeordnet und entgegengesetzt zu der ersten Handhabungsfläche 15 in Axialrichtung A orientiert. Zur Pumpbewegung des Faltenbalgs 8 können die Handhabungsflächen 15, 16 beispielsweise zwischen den Fingern einer Hand in Axialrichtung A aufeinander zubewegt werden, wobei der Faltenbalg 8 zusammengedrückt und das Ausgleichsvolumen 9 dementsprechend verringert wird.

Der Faltenbalg 8 bewirkt aufgrund seiner elastischen Gestaltgebung und/oder der vorliegenden Auswahl des Werkstoffs W eine der vorbeschriebenen Pumpbewegung entgegengerichtete Federkraft F` (Fig. 1). Die Federkraft F` ist in Axialrichtung A orientiert. Anstelle einer solchen Gestaltung kann beispielsweise eine gesonderte Federeinrichtung und/oder ein gesondertes Federelement vorgesehen sein, das mit dem Faltenbalg 8 und/oder den Gehäuseteilen 13, 14 wirkverbunden sein kann.

Weiterhin ist vorliegend ein erstes Ventil 17 vorgesehen. Das erste Ventil 17 ist dem ersten Durchlass 4 zugeordnet und im Bereich des ersten Konnektorabschnitts 3 angeordnet. Das erste Ventil 17 ist zwischen einer Sperrstellung (vgl. Fig. 4) und einer Durchflussstellung (vgl. Fig. 3) überführbar, wobei der erste Durchlass 4 in der Sperrstellung fluiddicht von dem Ausgleichsvolumen 9 und damit auch von dem Fluidkanalabschnitt 10 getrennt ist. In der Durchflussstellung ist der erste Durchlass 4 demgegenüber fluidleitend mit dem Ausgleichsvolumen 9 und dem Fluidkanalabschnitt 10 verbunden.

Das erste Ventil ist vorliegend in Form eines manuell betätigbaren Sperrventils 17 ausgebildet. Das Sperrventil 17 weist einen Betätigungsgriff 18 auf. Die diesbezügliche Gestaltung ist als rein exemplarisch zu verstehen.

Weiter weist die Fluidübertragungsvorrichtung 1 ein zweites Ventil 19 auf, das ebenfalls zwischen einer Sperrstellung (Fig. 3) und einer Durchflussstellung (Fig. 4) überführbar ist. In der Sperrstellung ist das Ausgleichsvolumen 9 fluiddicht von dem zweiten Durchlass 6 getrennt. Demgegenüber ist das Ausgleichsvolumen 9 in der Durchflussstellung fluidleitend mit dem zweiten Durchlass 6 verbunden.

Das zweite Ventil ist vorliegend in Form eines Druckventils 19 ausgebildet, das in Abhängigkeit eines in dem Ausgleichsvolumen 9 vorherrschenden Drucks selbsttätig zwischen der Sperrstellung und der Durchflussstellung überführbar ist. Das Druckventil 19 ist vorliegend membranartig gestaltet und weist einen schlitzartigen Durchlass 20 auf, der in Abhängigkeit des besagten Drucks in dem Ausgleichsvolumen 9 infolge einer druckbedingten elastischen Deformation freigegeben oder verschlossen ist.

In einem zeichnerisch nicht näher dargestellten Auslieferungszustand der Fluidübertragungsvorrichtung 1 nehmen sowohl das Sperrventil 17 als auch das Druckventil 19 die jeweilige Sperrstellung ein. Zudem ist das Ausgleichsvolumen 9 mit einem nicht näher bezeichneten sterilen Gas befüllt. Durch die Befüllung mit dem sterilen Gas, das beispielsweise sterile Luft sein kann, wird vermieden, dass der Ausgleichsbehälter 8 in unerwünschter Weise zusammengedrückt wird.

Anhand der Fig. 3 und 4 wird die Funktionsweise der Fluidübertragungsvorrichtung 1 bei einem Zuführen der Medikamentenflüssigkeit F in den Flüssigkeitsbehälter 2 näher beschrieben.

Hierzu wird ausgehend von der anhand Fig. 2 ersichtlichen Konfiguration zunächst eine mit der Medikamentenflüssigkeit F befüllte Spritze auf die vorbeschriebene Weise fluiddicht mit dem ersten Konnektorabschnitt 3 verbunden. Der zweite Konnektorabschnitt in Form des Einstechdorns 5 ist fluiddicht in den Flüssigkeitsbehälter 2 eingestoßen, wobei der zweite Durchlass 6 fluidleitend in das Innere des Flüssigkeitsbehälters 2 reicht. Hierbei nimmt das Sperrventil 17 - abweichend von der anhand Fig. 2 ersichtlichen Konfiguration - zunächst die Sperrstellung ein und wird manuell mittels einer Betätigung des Betätigungsgriffs 18 in die Durchflussstellung (Fig. 3) überführt. Hiernach kann die Medikamentenflüssigkeit F mittels einer grundsätzlich bekannten Betätigung der medizinischen Spritze durch den ersten Durchlass 4 in den Fluidkanal 7 eingespritzt werden und gelangt von diesem weiter in den Fluidkanalabschnitt 10 bzw. das Ausgleichsvolumen 9. Hierbei ist es vorteilhaft, wenn die Axialrichtung A der Fluidübertragungsvorrichtung 1 parallel zu dem Erdschwerevektor g orientiert ist. Eine solche Orientierung ist jedoch nicht zwingend notwendig. Durch das Zuführen der Medikamentenflüssigkeit F in das Ausgleichsvolumen 9 bildet sich ein anhand Fig. 3 ersichtlicher nicht näher bezeichneter Pegel der Medikamentenflüssigkeit F in dem Faltenbalg 8 aus. Das auf diese Weise zugeführte Volumen der Medikamentenflüssigkeit F führt zu einer Expansion des Faltenbalgs 8 in Axialrichtung A. Das Druckventil 19 ist vorliegend derart ausgelegt, dass der schlitzartige Durchlass 20 bei einem Einspritzen der Medikamentenflüssigkeit F zunächst verschlossen bleibt, so dass das Druckventil 19 seine Sperrstellung einnimmt.

Hiernach wird das Sperrventil 17 manuell in die Sperrstellung überführt (Fig. 4). Um die Medikamentenflüssigkeit F ausgehend von dem Ausgleichsvolumen 9 in den Flüssigkeitsbehälter 2 zu überführen, wird der Faltenbalg 8 mittels der vorbeschriebenen manuellen Betätigung der Handhabungsflächen 15, 16 in Axialrichtung A gestaucht. Hierdurch wird die Medikamentenflüssigkeit F infolge eines Druckanstiegs in dem Ausgleichsvolumen 9 durch den schlitzartigen Durchlass 20 und von dort weiter über den zweiten Durchlass 6 in den Flüssigkeitsbehälter 2 überführt. Mit anderen Worten ausgedrückt wird das Druckventil 19 infolge der manuellen Pumpbewegung des Faltenbalgs 8 in seine Durchflussstellung überführt.

Das auf diese Weise dem Flüssigkeitsbehälter 2 zugeführte Volumen der Medikamentenflüssigkeit F führt naturgemäß zu einer Verdrängung eines nicht näher bezeichneten Gasvolumens. Das Gasvolumen strömt hierbei über eine fluidleitende Verbindung in das Ausgleichsvolumen 9 und wird aufgrund der fluiddichten Gestaltung des Ausgleichsvolumens 9 hermetisch in diesem aufgenommen, ohne dass es zu einer Kontamination der Umgebung kommt.

Die fluidleitende Verbindung zwischen dem Ausgleichsvolumen 9 und dem Flüssigkeitsbehälter 2 ist vorliegend mittels eines dritten Durchlasses 21 realisiert, der in Form eines Lumens durch den Einstechdorn 5 erstreckt ist. Eine solche Gestaltung ist jedoch nicht zwingend.

Die manuelle Pumpbewegung kann wiederholt werden, bis die gesamte Medikamentenflüssigkeit F von dem Ausgleichsvolumen 9 in den Flüssigkeitsbehälter 2 überführt ist.

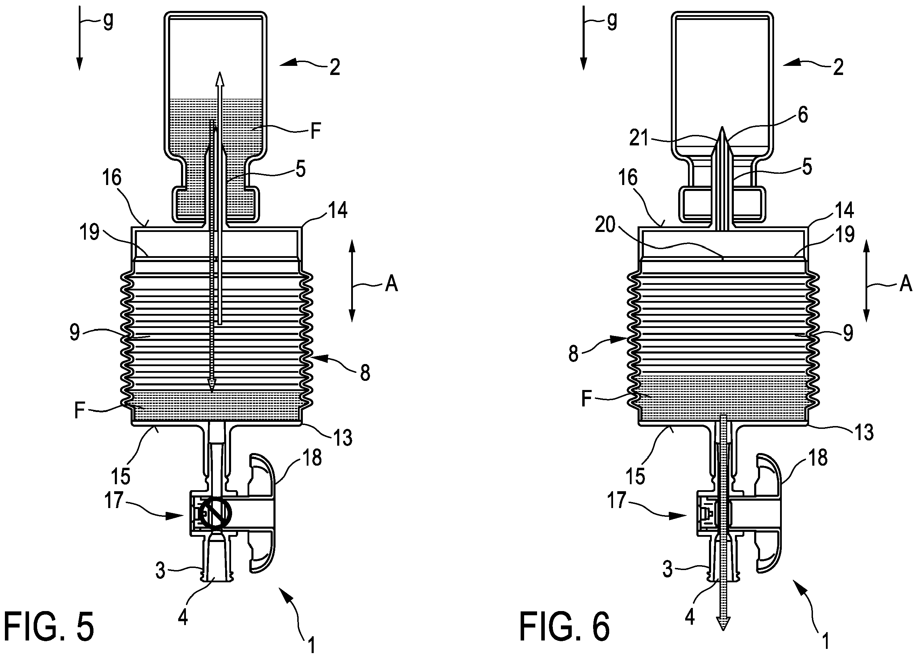

Anhand der Fig. 5 und 6 ist die Funktionsweise der Fluidübertragungsvorrichtung 1 bei einer Entnahme der Medikamentenflüssigkeit F aus dem Flüssigkeitsbehälter 2 ersichtlich.

Dabei wird ausgehend von einer Konfiguration, in der der Flüssigkeitsbehälter 2 mit der Medikamentenflüssigkeit F befüllt ist, zunächst der zweite Konnektorabschnitt 5 fluiddicht mit dem Flüssigkeitsbehälter 2 verbunden. Vorliegend wird hierzu der Einstechdorn 5 auf die vorbeschriebene Weise in den Flüssigkeitsbehälter 2 eingestochen. Das Sperrventil 17 nimmt hierbei seine Sperrstellung ein. Die medizinische Spritze kann hierbei bereits fluiddicht mit dem ersten Konnektorabschnitt 3 verbunden sein. Alternativ kann diese Verbindung auch erst zu einem späteren Zeitpunkt erfolgen.

Die Entnahme der Medikamentenflüssigkeit F erfolgt vorliegend - ebenso wie das anhand der Fig. 3 und 4 verdeutlichte Zuführen - zweistufig. Zunächst wird die Fluidübertragungsvorrichtung 1 zusammen mit dem daran befindlichen Flüssigkeitsbehälter 2 entsprechend Fig. 5 orientiert, so dass der Einstechdorn 5 entgegen dem Erdschwerevektor g vertikal nach oben zeigt. Sodann wird der Faltenbalg 8 manuell in Axialrichtung A zusammengedrückt. Hierdurch wird das Ausgleichsvolumen 9 verringert und das Druckventil 19 wird infolge des auf diese Weise hervorgerufenen Druckanstiegs in die Durchflussstellung überführt. Durch die Verringerung des Ausgleichsvolumens 9 kann ein darin befindliches Gasvolumen über den schlitzartigen Durchlass 20 und von dort weiter durch den zweiten Durchlass 6 und/oder den dritten Durchlass 21 in den Flüssigkeitsbehälter 2 gelangen. Die in diesem befindliche Medikamentenflüssigkeit F wird hierdurch verdrängt und gelangt über den zweiten Durchlass 6 oder den dritten Durchlass 21 und den schlitzartigen Durchlass 20 in das Ausgleichsvolumen 9. Hierdurch bildet sich ein anhand Fig. 5 ersichtlicher Pegel der Medikamentenflüssigkeit F in dem Faltenbalg 8 aus. Dieser Vorgang kann so oft wiederholt werden, bis der Flüssigkeitsbehälter 2 im Wesentlichen vollständig entleert ist.

Sofern noch nicht geschehen, wird die medizinische Spritze fluiddicht mit dem ersten Konnektorabschnitt 3 verbunden, das Sperrventil 17 wird manuell in seine Durchflussstellung überführt und die Medikamentenflüssigkeit F kann mittels einer entsprechenden Betätigung der medizinischen Spritze aus dem Ausgleichsvolumen 9 abgesaugt werden. Hierbei verringert sich das Ausgleichsvolumen 9 dementsprechend aufgrund des abgesaugten Volumens der Medikamentenflüssigkeit F.

Hierbei ist besonders vorteilhaft, dass die Medikamentenflüssigkeit F in der anhand Fig. 6 ersichtlichen Konfiguration ohne weiteres zurück in das Ausgleichsvolumen 9 gespritzt werden kann. Hierdurch können etwaige in der medizinischen Spritze vorhandene Luftblasen oder überschüssige Medikamentenflüssigkeit F auf einfache Weise und ohne die Fluidübertragungsvorrichtung 1 zuvor in die anhand der Fig. 3 und 4 ersichtliche Orientierung wenden zu müssen aus der medizinischen Spritze entfernt werden.

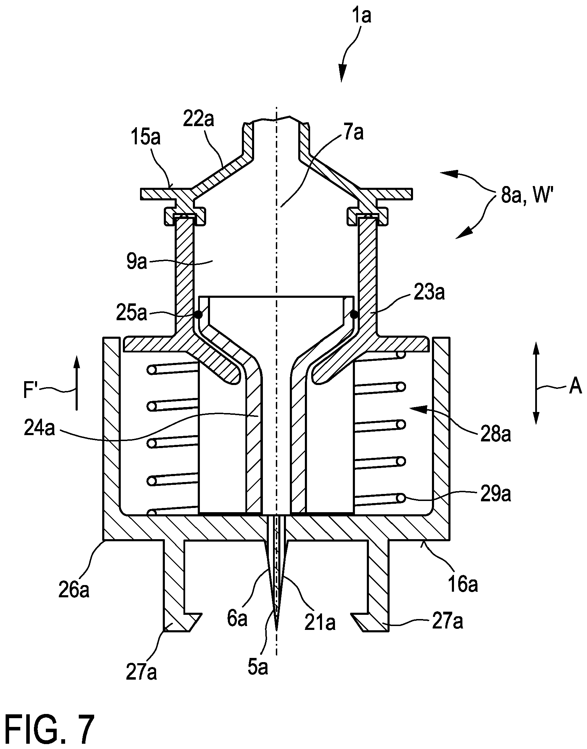

Anhand Fig. 7 ist eine weitere Ausführungsform einer erfindungsgemäßen Fluidübertragungsvorrichtung 1a ersichtlich. Die Fluidübertragungsvorrichtung 1a nach Fig. 7 entspricht im Wesentlichen der zuvor anhand der Fig. 1 bis 6 beschriebenen Fluidübertragungsvorrichtung 1. Zur Vermeidung von Wiederholungen wird daher auf die Offenbarung im Zusammenhang mit den Fig. 1 bis 6 verwiesen. Nachfolgend wird lediglich auf die wesentlichen Unterschiede der Ausführungsform nach Fig. 7 eingegangen. Dabei sind funktions- und/oder baugleiche Abschnitte und Teile der Ausführungsform nach Fig. 7 mit gleichen Bezugszeichenziffern unter Hinzufügung des Kleinbuchstabens a versehen.

Die Fluidübertragungsvorrichtung 1a unterscheidet sich in erster Linie durch die Ausgestaltung des Ausgleichsbehälters 8a von der Fluidübertragungsvorrichtung 1. Der Ausgleichsbehälter 8a weist vorliegend mehrere aus einem formstabilen Werkstoff W' gefertigte Behälterteile 22a, 23a und 24a auf. Die Behälterteile 22a, 23a, 24a umgrenzen das Ausgleichsvolumen 9a. Zur Volumenveränderung des Ausgleichsvolumens 9a sind die Behälterteile 22a, 23a relativ zu dem Behälterteil 24a verschieblich.

Dabei ist das erste Behälterteil 22a nach Art eines Deckels in Axialrichtung A stirnendseitig fluiddicht und fest mit dem zweiten Behälterteil 23a zusammengefügt. Als Fügeverbindung ist vorliegend eine Ultraschallverschweißung vorgesehen, was jedoch nicht zwingend ist. Das dritte Behälterteil 24a weist einends stirnendseitig eine kelchartige Erweiterung auf, die fluiddicht an einer nicht näher bezeichneten Innenwandung des zweiten Behälterteils 23a anliegt und relativ zu dieser gleitbeweglich ist. Zur fluiddichten Abdichtung zwischen dem zweiten Behälterteil 23a und dem dritten Behälterteil 24a ist vorliegend ein Dichtelement in Form eines O-Rings 25a vorgesehen.

Andernends ist das dritte Behälterteil 24a fluiddicht und fest mit einer Rasteinrichtung 26a zusammengefügt. Die Rasteinrichtung 26a ist aus einem formstabilen nicht näher bezeichneten Werkstoff gefertigt und ist zur unlösbaren Rastverbindung der Fluidübertragungsvorrichtung 1 mit einem Flüssigkeitsbehälter entsprechend dem Flüssigkeitsbehälter 2 gemäß den Fig. 1 bis 6 vorgesehen. Zu diesem Zweck weist die Rasteinrichtung 26a vorliegend mehrere Rastelemente 27a auf, die zur formschlüssigen Verbindung mit einem Halsbereich des Flüssigkeitsbehälters 2 vorgesehen sind.

Der zweite Konnektorabschnitt ist vorliegend wiederum in Form eines Einstechdorns 5a ausgebildet. Der Einstechdorn 5a ist einstückig an der Rasteinrichtung 26a ausgebildet und ragt in Axialrichtung A unterseitig von einem nicht näher bezeichneten becherartigen Abschnitt der Rasteinrichtung 26a ab.

Der Ausgleichsbehälter 8a ist wiederum manuell pumpbeweglich. Um eine erleichterte Pumpbeweglichkeit zu erreichen, sind Handhabungsflächen 15a, 16a vorgesehen. Die erste Handhabungsfläche 15a ist oberseitig an dem ersten Behälterteil 22a angeordnet. Die zweite Handhabungsfläche 16a ist unterseitig an der Rasteinrichtung 26a angeordnet.

Um eine vereinfachte Rückstellung des Ausgleichsbehälters 8a bei einer manuellen Pumpbewegung zu erreichen, ist vorliegend eine mit dem Ausgleichsbehälter 8a wirkverbundene Federeinrichtung 28a vorgesehen. Die Federeinrichtung 28a weist ein Federelement in Form einer auf Druck zu belastenden Wendelfeder 29a auf. Die Wendelfeder 29a ist einends an dem becherartigen Abschnitt der Rasteinrichtung 26a abgestützt. Andernends ist die Wendelfeder 29a unterseitig an dem zweiten Behälterteil 23a abgestützt.

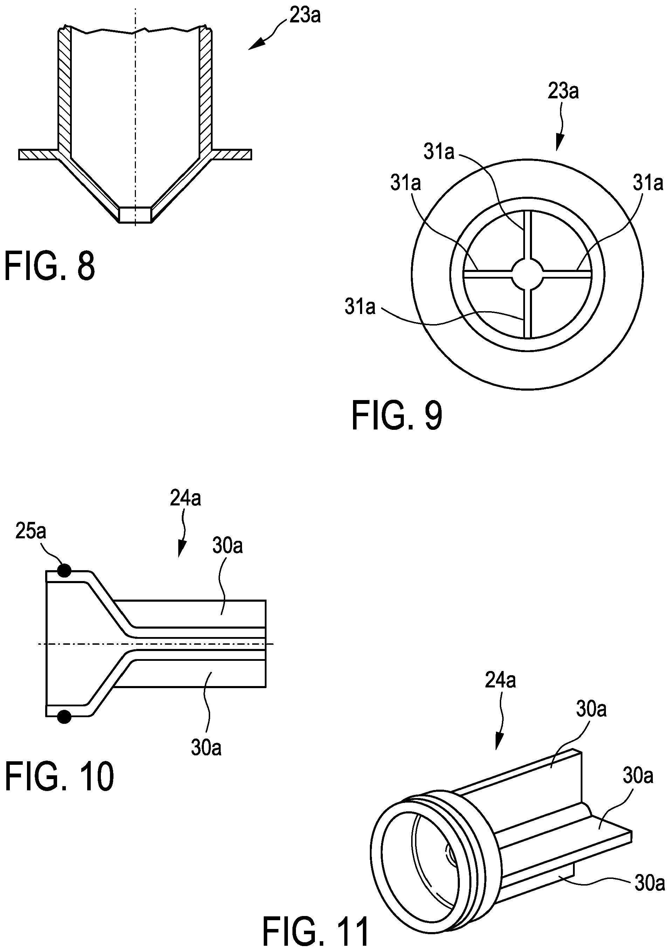

Anhand der Fig. 8 bis 11 sind weitere Einzelheiten der spezifischen Gestaltung der Behälterteile 23a und 24a ersichtlich.

Der dritte Behälterteil 24a weist insbesondere mehrere flügelartig in Radialrichtung abstehende Flügelabschnitte 30a auf. Vorliegend sind vier Flügelabschnitte 30a vorgesehen, die jeweils um 90° versetzt in Umfangsrichtung des dritten Behälterteils 24a angeordnet sind. Anhand Fig. 9 ist ersichtlich, dass das zweite Behälterteil 23a eine hierzu komplementäre Gestaltung aufweist, wobei mehrere in Umfangsrichtung versetzt angeordnete und in Radialrichtung erstreckte Schlitze 31a zur Aufnahme jeweils eines der Flügelabschnitte 30a vorgesehen sind.

## Patentansprüche

1. Medizinische Fluidübertragungsvorrichtung (1, 1a) zum fluiddicht abgeschlossenen Übertragen einer gesundheitsgefährdenden Medikamentenflüssigkeit (F), aufweisend
- einen ersten Konnektorabschnitt (3), der einen ersten Durchlass (4) aufweist und zur fluiddichten Verbindung mit einer medizinischen Spritze vorgesehen ist,
- einen zweiten Konnektorabschnitt (5, 5a), der einen zweiten Durchlass (6, 6a) und einen dritten Durchlass (21) aufweist und zur fluiddichten Verbindung mit einem medizinischen Flüssigkeitsbehälter (2) vorgesehen ist,
- einen zwischen dem ersten Durchlass (4) und dem zweiten Durchlass (6, 6a) erstreckten Fluidkanal (7, 7a), der - in einem mit der Spritze und dem Flüssigkeitsbehälter (2) verbundenen Zustand der Fluidübertragungsvorrichtung (1, 1a) - eine fluidleitende Übertragung der Medikamentenflüssigkeit (F) zwischen der Spritze und dem Flüssigkeitsbehälter (2) gestattet, und
- einen Ausgleichsbehälter (8, 8a) mit einem fluiddichten und volumenveränderlichen Ausgleichsvolumen (9, 9a), das - im verbundenen Zustand der Fluidübertragungsvorrichtung (1, 1a) - mittels des dritten Durchlasses (21) fluidleitend mit dem Flüssigkeitsbehälter (2) verbunden ist, und das zur Aufnahme eines bei der Übertragung der Medikamentenflüssigkeit (F) aus dem Flüssigkeitsbehälter (2) verdrängten Gasvolumens eingerichtet ist,
- **dadurch gekennzeichnet, dass** der erste Durchlass (4) und der zweite Durchlass (6, 6a) über das Ausgleichsvolumen (9, 9a) fluidleitend miteinander verbunden sind, wobei das Ausgleichsvolumen (9, 9a) einen Fluidkanalabschnitt (10, 10a) des Fluidkanals (7, 7a) bildet.

2. Medizinische Fluidübertragungsvorrichtung (1, 1a) nach Anspruch 1, **dadurch gekennzeichnet, dass** der erste Konnektorabschnitt (3) und der zweite Konnektorabschnitt (5, 5a) an einander gegenüberliegenden Stirnendbereichen (11, 12) des Ausgleichsbehälters (8, 8a) angeordnet sind.

3. Medizinische Fluidübertragungsvorrichtung (1) nach Anspruch 1 oder 2, **dadurch gekennzeichnet, dass** der Ausgleichsbehälter (8) aus einem elastischen Werkstoff (W) gefertigt ist und/oder eine elastische Gestaltgebung aufweist, wodurch das Ausgleichsvolumen (9) zur Volumenänderung elastisch expandierbar und elastisch kontrahierbar ist.

4. Medizinische Fluidübertragungsvorrichtung (1) nach Anspruch 3, **dadurch gekennzeichnet, dass** der Ausgleichsbehälter ein Faltenbalg (8) ist.

5. Medizinische Fluidübertragungsvorrichtung (1a) nach Anspruch 1 oder 2, **dadurch gekennzeichnet, dass** der Ausgleichsbehälter (8a) wenigstens zwei aus einem formstabilen Werkstoff (W') gefertigte Behälterteile (22a, 23a, 24a) aufweist, die das Ausgleichsvolumen (9a) umgrenzen und die zur Volumenänderung des Ausgleichsvolumens (9a) zueinander relativbeweglich sind.

6. Medizinische Fluidübertragungsvorrichtung (1, 1a) nach einem der vorhergehenden Ansprüche, **dadurch gekennzeichnet, dass** der Ausgleichsbehälter (8, 8a) zur Übertragung der Medikamentenflüssigkeit (F) zwischen dem ersten Durchlass (4) und dem zweiten Durchlass (6, 6a) manuell pumpbeweglich ist.

7. Medizinische Fluidübertragungsvorrichtung (1, 1a) nach Anspruch 6, **dadurch gekennzeichnet, dass** mit dem Ausgleichsbehälter (8, 8a) wirkverbundene Handhabungsflächen (15, 15a, 16, 16a) vorgesehen sind, die zur manuellen Pumpbewegung des Ausgleichsbehälters (8, 8a) relativ zueinander verlagerbar sind.

8. Medizinische Fluidübertragungsvorrichtung (1a) nach Anspruch 6 oder 7, **dadurch gekennzeichnet, dass** eine mit dem Ausgleichsbehälter (8a) wirkverbundene Federeinrichtung (28a) vorgesehen ist, die eine das Ausgleichsvolumen (9a) expandierende Federkraft (F') bewirkt.

9. Medizinische Fluidübertragungsvorrichtung (1) nach einem der vorhergehenden Ansprüche, **dadurch gekennzeichnet, dass** ein zwischen einer Sperrstellung und einer Durchflussstellung überführbares erstes Ventil (17) vorgesehen ist, mittels dessen der erste Durchlass (4) in der Sperrstellung fluiddicht von dem Ausgleichsvolumen (9) getrennt ist und in der Durchflussstellung fluidleitend mit dem Ausgleichsvolumen (9) verbunden ist.

10. Medizinische Fluidübertragungsvorrichtung (1) nach Anspruch 9, **dadurch gekennzeichnet, dass** das erste Ventil ein zur manuellen Betätigung eingerichtetes Sperrventil (17) ist.

11. Medizinische Fluidübertragungsvorrichtung (1) nach einem der vorhergehenden Ansprüche, **dadurch gekennzeichnet, dass** ein zwischen einer Sperrstellung und einer Durchflussstellung überführbares zweites Ventil (19) vorgesehen ist, mittels dessen das Ausgleichsvolumen (9) in der Sperrstellung fluiddicht von dem zweiten Durchlass (6) getrennt ist und in der Durchflussstellung fluidleitend mit dem zweiten Durchlass (6) verbunden ist.

12. Medizinische Fluidübertragungsvorrichtung (1) nach Anspruch 11, **dadurch gekennzeichnet, dass** das zweite Ventil ein Druckventil (19) ist, das in Abhängigkeit eines in dem Ausgleichsvolumen (9) anliegenden Drucks selbsttätig zwischen der Sperrstellung und der Durchflussstellung überführbar ist.

13. Medizinische Fluidübertragungsvorrichtung (1) nach Anspruch 9 und Anspruch 11, **dadurch gekennzeichnet, dass** das erste Ventil (17) und das zweite Ventil (19) in einem Auslieferungszustand der Fluidübertragungsvorrichtung (1) jeweils die Sperrstellung einnehmen, und dass das Ausgleichsvolumen (9) mit einem sterilen Gas befüllt ist.

14. Medizinische Fluidübertragungsvorrichtung (1a) nach einem der vorhergehenden Ansprüche, **dadurch gekennzeichnet, dass** eine dem zweiten Konnektorabschnitt (5a) zugeordnete Rasteinrichtung (26a) vorgesehen ist, die eine unlösbare Rastverbindung mit dem Medikamentenbehälter (2) gestattet.

## Claims

1. Medical fluid transfer device (1, 1a) for the fluid-tightly sealed transfer of a medical fluid (F) which is hazardous to health, comprising
a first connector section (3), which has a first passage (4) and is provided for fluid-tight connection to a medical syringe,
a second connector section (5, 5a), which has a second passage (6, 6a) and a third passage (21) and is provided for fluid-tight connection to a medical fluid container (2),
a fluid channel (7, 7a) extending between the first passage (4) and the second passage (6, 6a), which - in a state of the fluid transfer device (1, 1a) connected to the syringe and the medical fluid container (2) - allows a fluid-conducting transfer of the medical fluid (F) between the syringe and the medical fluid container (2), and
a compensation container (8, 8a) with a fluid-tight and variable-volume compensation volume (9, 9a) which - in the connected state of the fluid transfer device (1, 1a) - is connected to the medical fluid container (2) in a fluid-conducting manner by means of the third passage (21), and which is designed to receive a volume of gas displaced from the medical fluid container (2) during the transfer of the medical fluid (F),
**characterized in that** the first passage (4) and the second passage (6, 6a) are connected to one another in a fluid-conducting manner via the compensation volume (9, 9a), the compensation volume (9, 9a) forming a fluid channel section (10, 10a) of the fluid channel (7, 7a).

2. Medical fluid transfer device (1, 1a) according to claim 1, **characterized in that** the first connector section (3) and the second connector section (5, 5a) are arranged at opposite end regions (11, 12) of the compensation container (8, 8a).

3. Medical fluid transfer device (1) according to claim 1 or 2, **characterized in that** the compensation container (8) is made of an elastic material (W) and/or has an elastic shape, whereby the compensation volume (9) is elastically expandable and elastically contractible for volume change.

4. Medical fluid transfer device (1) according to claim 3, **characterized in that** the compensation container is a bellows (8).

5. Medical fluid transfer device (1a) according to claim 1 or 2, **characterized in that** the compensation container (8a) has at least two container parts (22a, 23a, 24a) which are made of a dimensionally stable material (W), which delimit the compensation volume (9a) and which are movable relative to one another in order to change the volume of the compensation volume (9a).

6. Medical fluid transfer device (1, 1a) according to one of the preceding claims, **characterized in that** the compensation container (8, 8a) for transferring the medicament fluid (F) between the first passage (4) and the second passage (6, 6a) is manually pumpable.

7. Medical fluid transfer device (1, 1a) according to claim 6, **characterized in that** handling surfaces (15, 15a, 16, 16a) are provided which are operatively connected to the compensation container (8, 8a) and can be displaced relative to one another for the manual pumping movement of the compensation container (8, 8a).

8. Medical fluid transfer device (1a) according to claim 6 or 7, **characterized in that** a spring device (28a) operatively connected to the compensation container (8a) is provided, which causes a spring force (F') expanding the compensation volume (9a).

9. Medical fluid transfer device (1) according to one of the preceding claims, **characterized in that** a first valve (17) is provided which can be transferred between a blocking position and a flow-through position and by means of which the first passage (4) is separated from the compensation volume (9) in a fluid-tight manner in the blocking position and is connected to the compensation volume (9) in a fluid-conducting manner in the flow-through position.

10. Medical fluid transfer device (1) according to claim 9, **characterized in that** the first valve is a shut-off valve (17) being configured for manual actuation.

11. Medical fluid transfer device (1) according to one of the preceding claims, **characterized in that** a second valve (19) is provided which can be transferred between a blocking position and a flow-through position and by means of which the compensation volume (9) is separated from the second passage (6) in a fluid-tight manner in the blocking position and is connected to the second passage (6) in a fluid-conducting manner in the flow-through position.

12. Medical fluid transfer device (1) according to claim 11, **characterized in that** the second valve is a pressure valve (19) which can be automatically transferred between the blocking position and the flow-through position as a function of a pressure present in the compensation volume (9).

13. Medical fluid transfer device (1) according to claim 9 and claim 11, **characterized in that** the first valve (17) and the second valve (19) each assume the blocking position in a delivery state of the fluid transfer device (1), and **in that** the compensation volume (9) is filled with a sterile gas.

14. Medical fluid transfer device (1a) according to one of the preceding claims, **characterized in that** a latching device (26a) associated with the second connector section (5a) is provided, which permits an undetachable latching connection with the medical fluid container (2).

## Revendications

1. Dispositif de transfert de fluide médical (1, 1a) pour le transfert étanche au fluide d'un fluide médical (F) dangereux pour la santé, comprenant
une première partie de connecteur (3) qui comprend un premier passage (4) et qui est destinée à être connectée de manière étanche au fluide à une seringue médicale,
une deuxième partie de connecteur (5, 5a) comprenant un deuxième passage (6, 6a) et un troisième passage (21) et prévue pour une connexion étanche au fluide avec un réservoir de fluide médical (2),
un canal de fluide (7, 7a) s'étendant entre le premier passage (4) et le deuxième passage (6, 6a), qui - dans un état du dispositif de transfert de fluide (1, 1a) relié à la seringue et au réservoir de fluid médical (2) - permet un transfert par conduction de fluide du fluide médical (F) entre la seringue et le réservoir de fluide médical (2), et
un réservoir de compensation (8, 8a) avec un volume de compensation (9, 9a) étanche au fluide et à volume variable, qui - à l'état connecté du dispositif de transfert de fluide (1, 1a) - est relié au réservoir de fluide médical (2) de manière à conduire le fluide au moyen du troisième passage (21), et qui est aménagé pour recevoir un volume de gaz déplacé hors du réservoir de fluide médical (2) lors du transfert du fluide médical (F),
**caractérisé en ce que** le premier passage (4) et le deuxième passage (6, 6a) sont reliés l'un à l'autre de manière à conduire le fluide par l'intermédiaire du volume de compensation (9, 9a), le volume de compensation (9, 9a) formant une section de canal de fluide (10, 10a) du canal de fluide (7, 7a).

2. Dispositif de transfert de fluide médical (1, 1a) selon la revendication 1, **caractérisé en ce que** la première partie de connecteur (3) et la deuxième partie de connecteur (5, 5a) sont disposées sur des zones d'extrémité opposées (11, 12) du réservoir de compensation (8, 8a).

3. Dispositif de transfert de fluide médical (1) selon la revendication 1 ou 2, **caractérisé en ce que** le réservoir de compensation (8) est fabriqué en un matériau élastique (W) et/ou présente une configuration élastique, ce qui permet au volume de compensation (9) de se dilater élastiquement et de se contracter élastiquement pour modifier son volume.

4. Dispositif de transfert de fluide médical (1) selon la revendication 3, **caractérisé en ce que** le réservoir de compensation est un soufflet (8).

5. Dispositif de transfert de fluide médical (1a) selon la revendication 1 ou 2, **caractérisé en ce que** le réservoir de compensation (8a) présente au moins deux parties de réservoir (22a, 23a, 24a) fabriquées dans un matériau indéformable (W), qui délimitent le volume de compensation (9a) et qui sont mobiles l'une par rapport à l'autre pour modifier le volume du volume de compensation (9a).

6. Dispositif de transfert de fluide médical (1, 1a) selon l'une quelconque des revendications précédentes, **caractérisé en ce que** le réservoir compensation (8, 8a) est mobile par pompage manuel pour transférer le fluide médical (F) entre le premier passage (4) et le deuxième passage (6, 6a).

7. Dispositif de transfert de fluide médical (1, 1a) selon la revendication 6, **caractérisé en ce qu'**il est prévu des surfaces de manipulation (15, 15a, 16, 16a) en liaison active avec le réservoir de compensation (8, 8a), qui peuvent être déplacées les unes par rapport aux autres pour le mouvement de pompage manuel du réservoir de compensation (8, 8a).

8. Dispositif de transfert de fluide médical (1a) selon la revendication 6 ou 7, **caractérisé en ce qu'**il est prévu un dispositif à ressort (28a) en liaison active avec le récipient de compensation (8a), qui provoque une force de ressort (F') dilatant le volume de compensation (9a).

9. Dispositif de transfert de fluide médical (1) selon l'une des revendications précédentes, **caractérisé en ce qu'**il est prévu une première vanne (17) pouvant être transférée entre une position de blocage et une position de débit, au moyen de laquelle le premier passage (4) est séparé du volume d'équilibrage (9) de manière étanche aux fluides dans la position de blocage et est relié au volume de compensation (9) de manière à conduire les fluides dans la position de débit.

10. Dispositif de transfert de fluide médical (1) selon la revendication 9, **caractérisé en ce que** la première vanne est une vanne d'arrêt (17) adaptée pour être actionnée manuellement.

11. Dispositif de transfert de fluide médical (1) selon l'une des revendications précédentes, **caractérisé en ce qu'**il est prévu une deuxième vanne (19) pouvant être transférée entre une position de blocage et une position de débit, au moyen de laquelle le volume de compensation (9) est séparé du deuxième passage (6) de manière étanche au fluide dans la position de blocage et est relié au deuxième passage (6) de manière à conduire le fluide dans la position de débit.

12. Dispositif de transfert de fluide médical (1) selon la revendication 11, **caractérisé en ce que** la deuxième vanne est une vanne de pression (19) qui peut être transférée automatiquement entre la position de blocage et la position de débit en fonction d'une pression appliquée dans le volume de compensation (9).

13. Dispositif de transfert de fluide médical (1) selon la revendication 9 et la revendication 11, **caractérisé en ce que** la première vanne (17) et la deuxième vanne (19) occupent chacune la position de blocage dans un état de livraison du dispositif de transfert de fluide (1), et **en ce que** le volume de compensation (9) est rempli d'un gaz stérile.

14. Dispositif de transfert de fluide médical (1a) selon l'une quelconque des revendications précédentes, **caractérisé en ce qu'**il est prévu un dispositif d'encliquetage (26a) associé au deuxième partie de connecteur (5a) et permettant une liaison par encliquetage indémontable avec le réservoir de fluide médical (2).
